# EUROPEAN PATENT SPECIFICATION

(11) **EP 3 677 305 B1**
(45) Date of publication and mention of the grant of the patent: **13.11.2024**
(21) Application number: 19150430.7
(22) Date of filing: 04.01.2019
(51) Int. Cl.: A61M 39/24

(54) **ONE WAY INTRAVENOUS NEEDLE FREE VALVE INCLUDING A FLEXIBLE MEMBER**
INTRAVENÖSES NADELLOSES EINWEGVENTIL MIT EINEM FLEXIBLEN ELEMENT
SOUPAPE SANS AIGUILLE INTRAVEINEUSE À SENS UNIQUE COMPRENANT UN ÉLÉMENT FLEXIBLE

(43) Date of publication of application: 08.07.2020
(73) Proprietor: Asset Medikal Tasarim Sanayi ve Ticaret A.S., Basaksehir-Istanbul (TR)
(72) Inventor: TÜYSÜZ, Mehmet, 34303 Istanbul (TR); BASARAN, Ahmet Reha, 34303 Istanbul (TR)
(74) Representative: Sögüt, Onur Ömer

(56) References cited:
- WO-A1-2014/107436
- WO-A1-2014/153302
- DE-A1- 102010 022 410
- US-A- 4 188 978

## Description

### Technical Field of the Invention

The present invention relates to a one way intravenous needle free valve for medical use. In particular, the present invention relates to such valve with extended measures for prevention of reflux.

### Background of the Invention

EP 3023678 A1, US 201/0132680 A1, US 2016/0273665 A1, JP 2003198782 A, CN206034836U, US 2006/0173420 A1 and US 2014/0299212 A1 relate to one-way valves for use in medicine.

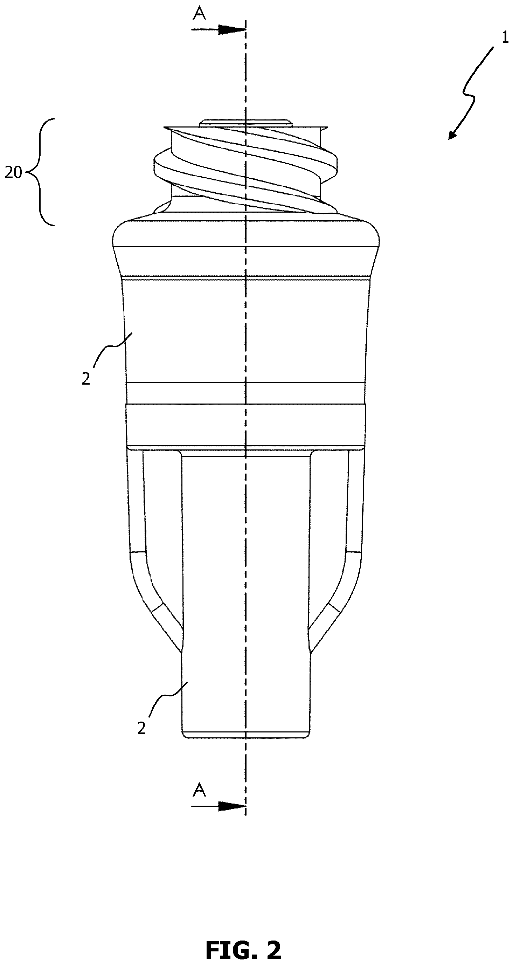

International publication WO 2014/107436 A1 relates to a check valve suitable for use with IV sets, providing an upstream filter for prevention of contamination from affecting the sealing of the check valve membrane. Fig.2, Fig.3, Fig.5, Fig.6, Fig.7A and Fig.8A of said international publication show that a flat membrane is employed in the check valve.

In general, fluid flow through the one-way valves in the current state of the art mainly occurs at flow paths between their respective flexible members and shells, and the flow is streamed through a substantially closed geometry, e.g. an annular space.

Geometrical details around such spaces are prone to cause dead volumes, purging of the fluids around which is difficult. Fluid flow around such dead zones inevitably occurs with lag and has unacceptably low linear velocity to allow effective cleaning of the wet surfaces of such valves.

This causes difficulty and inefficiency in washing/cleaning of such valves, and blood or drugs in liquid form can remain in such dead zones for unacceptably long times. Especially blood is known to be a fertile medium for rapid bacterial growth, and therefore dead zones are not wanted to be present in valves for medical use.

Therefore it is an intention to make improvements on flow paths inside one way intravenous needle free valve for medical use.

### Objects of the Invention

Primary object of the present invention is to overcome the abovementioned shortcomings of the prior art.

Another object of the present invention is to provide a one way intravenous needle free valve in which formation of dead volumes is minimized or eliminated.

A further object of the present invention is to provide a one way intravenous needle free valve with enhanced flow control.

Another object of the present invention is to provide a one way intravenous needle free valve without reflux.

### Summary of the Invention

The present invention proposes a one way intravenous needle free valve including a shell providing a fluid flow path along an axis between an inlet and an outlet; a flexible member placed between the inlet and the outlet and constrained throughout a circumferential edge thereof in a liquid-tight manner; an opening with a closed-geometry, in fluid communication with the inlet; the opening being aligned with a substantially impermeable central portion provided on the inlet side; wherein the member includes one or more holes around the central portion, providing a local fluid communication between the inlet side and the outlet side; and the member is shaped and sized to recline onto the opening such that the opening is substantially hermetically covered by the central portion when a fluid pressure at the outlet is equal to or greater than a fluid pressure at the inlet.

### Brief Description of the Drawings

The drawings, whose brief explanation is herewith provided, are solely intended for providing a better understanding of the present invention and are as such not intended to define the scope of protection or the context in which said scope is to be interpreted in the absence of the description.

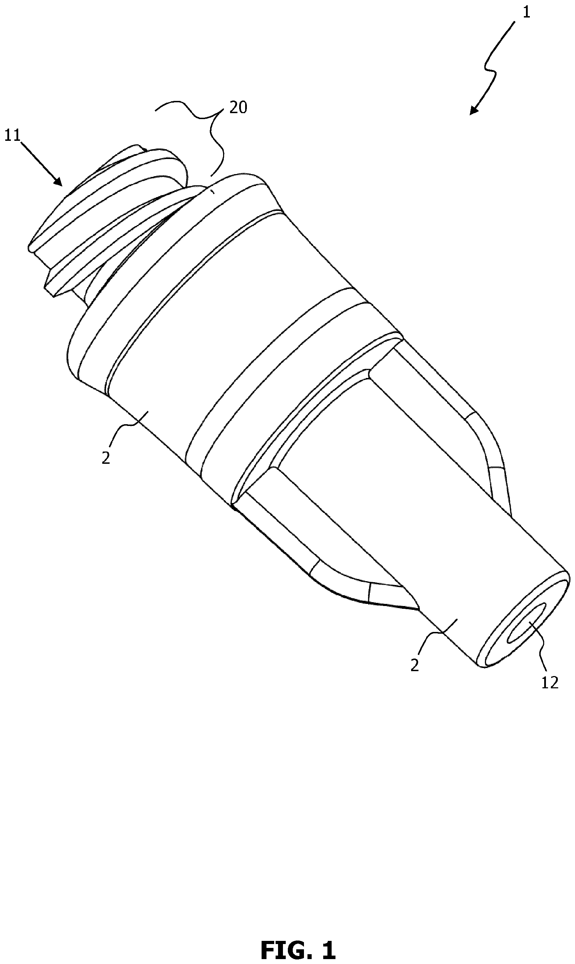

Fig.1 is a perspective view of an exemplary one way intravenous needle free valve according to the present invention.
Fig.2 is a side view of the valve shown in the Fig.1.
Fig.3 is A-A section view of the valve shown in the Fig. 2, emphasizing an exemplary positioning of a flexible member in said valve.
Fig.4(a) is a detail "D" from the Fig.3, showing a "normal" (i.e. closed) state of the valve; wherein fluid communication between an inlet and an outlet is blocked by the flexible member, an impermeable central portion of said flexible member being substantially hermetically reclined against a closed-geometry opening of the support, thus the opening is substantially sealed.
Fig.4(b) is the same detail showing an "activated" (i.e. open) state of the valve; wherein the fluid pressure at the inlet is substantially higher than the fluid pressure at the outlet, and accordingly:
   - the central portion of the flexible member is flexed towards the outlet and thus the central portion is at least partly moved away from the opening of the support, thereby said sealing of the opening is ceased;
   - and accordingly, fluid communication between the inlet and outlet is reversibly established.

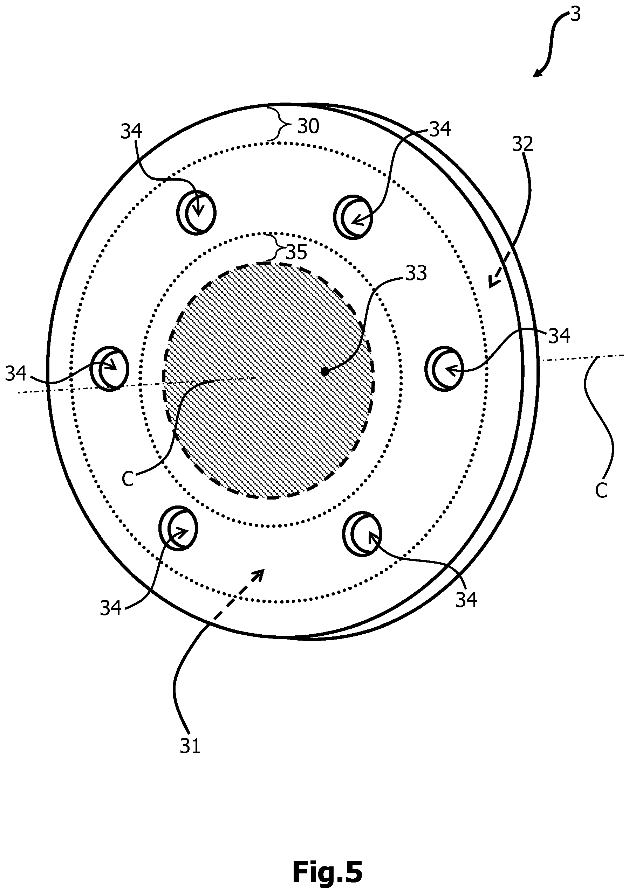

Fig.5 shows an inlet side perspective view of a flexible member suitable for being employed in the valve according to the present invention.

### Detailed Description of the Invention

Referring now the drawings outlined before, the present invention proposes a one way intravenous needle free valve (1) (hereinafter abbreviated as "valve") including a shell (2) which can be formed from a substantially rigid (i.e. non-flexible) material, providing a fluid flow path along an axis (C) between an inlet (11) and an outlet (12).

The valve (1) according to the present invention further comprises a flexible member (3) (hereinafter abbreviated as "member") placed between the inlet (11) and the outlet (12). The member (3) is constrained inside the shell (2), substantially throughout a circumferential edge (30) (hereinafter abbreviated as "edge") thereof in a liquid-tight manner. Thus, any fluid flow over said edge (30) is substantially prevented by continuous liquid-tight contact surfaces around the edge. As a result of said contact, the edge (30) defines an inlet side (31) of the member (3) in fluid communication with the inlet (11), and an outlet side (32) of the member (3) opposite to said inlet side, in fluid communication with the outlet (12).

The valve (1) includes an opening (41) with a closed-geometry, said opening (41) being in fluid communication with the inlet (11). The opening (41) is aligned with a substantially impermeable central portion (33) provided on the inlet side (31).

The member (3) includes one or more holes (34) around the central portion (33), providing a local fluid communication between the inlet side (31) and the outlet side (32).

The member (3) is shaped and sized to recline onto the opening (41) such that the opening (41) is substantially hermetically covered by the central portion (33) at a "normal state" of the valve (1).

Here, the term "normal state" refers to a case where a fluid pressure at the inlet (11) does not exceed a fluid pressure at the outlet (12), thus the central portion (33) is not subjected to a net fluid pressure, therefore the member (3) is not pushed away from the opening (41) (or, in other words, towards the outlet (12)).

As a result, as long as the normal state is maintained, the opening (41) is kept covered by the central portion (33), such that any fluid communication between the opening (41) and the outlet side (32) is substantially prevented. Thus in the normal state, any occurrence fluid leakage from the outlet (12) to the inlet (11) is likewise prevented.

With the above disclosed features of the valve (1) according to the present invention, the following behavior of the valve (1) is achieved:
- When the fluid pressure at the inlet (11) exceeds the fluid pressure at the outlet (12) sufficient to produce a net pressure to push the central portion (33) at least partly away from the opening (41) (thanks to the flexibility of the member (3)), the normal state ceases reversibly, and an "activated state" of the valve takes place (i.e. the valve is, reversibly, activated).
- As a result, the sealing of the opening (41) ceases, and a fluid communication between the opening (41) and the outlet side (32) is established through the one or more holes (34). Once said fluid communication is established, the fluid pressure at the inlet side (31) exceeding the fluid pressure at the outlet side (32) results in a one-way fluid flow from the inlet (11) towards the outlet (12), through the opening (41) and holes (34).
- The fluid pressure at the inlet (11) being greater than the fluid pressure at the outlet (12) inherently prevents any reflux (fluid leakage from the outlet to the inlet) in the activated state.
- When the fluid pressure at the outlet (12) gets equal to or higher than the fluid pressure at the inlet (11), the member (3) is no longer pushed away from the opening (41). As a result, the valve (1) turns back to its normal state where the opening (41) is closed by the central portion (33) thereby any fluid leakage from the outlet (12) to the inlet (11) is prevented. Thus, reflux is prevented in case of absence of a forced fluid stream through the valve (1).

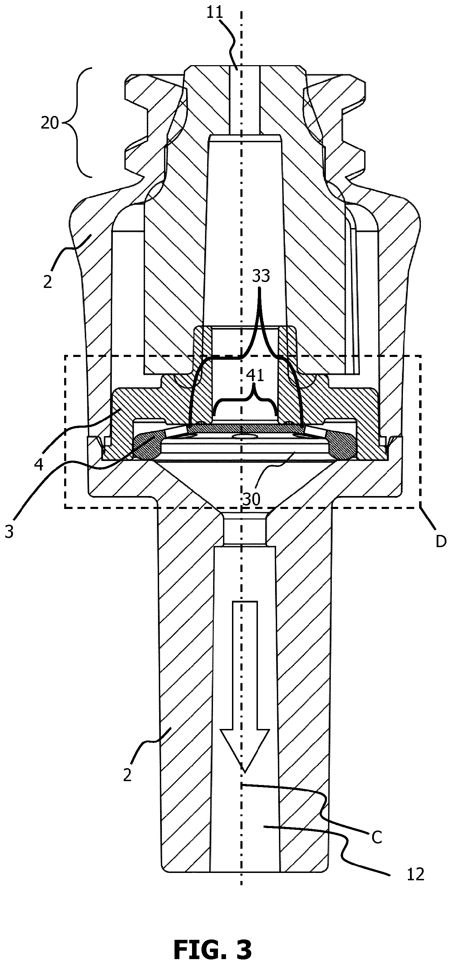

With the valve according to the present invention, fluid flow in the direction (emphasized with a bold arrow in Fig.3) towards the outlet (12), is only allowed in the case where the fluid pressure at the inlet (11) (thus at the opening (41)) is higher than the pressure at the outlet (12) (thus at the outlet side (32)).

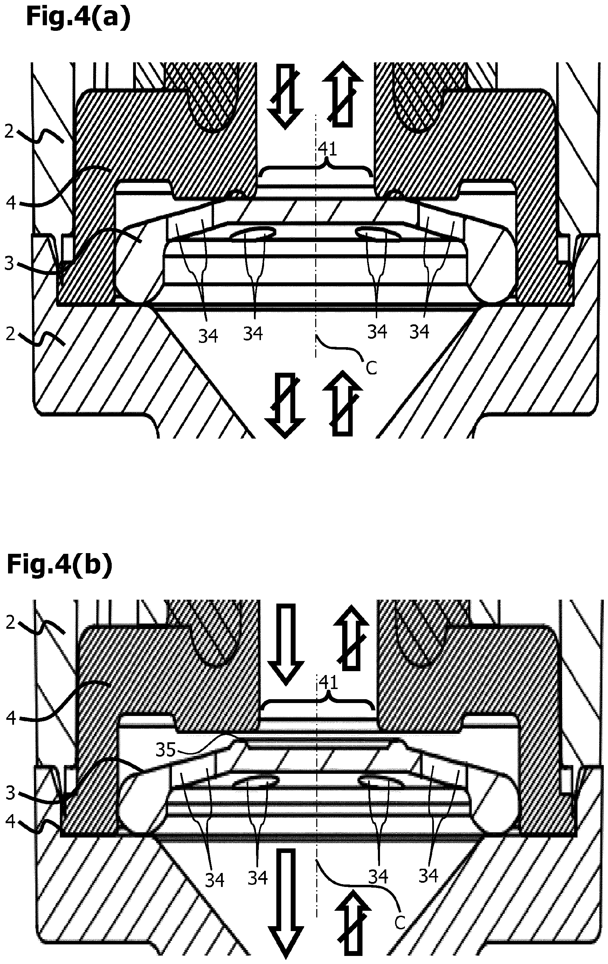

Fig.4(a) is a detail (D) taken from the Fig.3, showing a normal state of an exemplary valve (1) according to the present invention. Here, an exemplary way to ensure that the central portion (33) abuts/reclines against the opening (41) such that fluid communication between the opening (41) and the outlet side (32) is substantially prevented.

Fig.4(b) also shows the detail (D) from the Fig.3, showing an activated state of the same exemplary valve (1). Here, the central portion (33) is pushed away from the opening (41) by a net pressure force exerted by a fluid onto the central portion (33), which resulted in a reversible deformation of the member (3). Thus, fluid communication between the opening (41) and the outlet side (32) through the holes (34) is reversibly established.

In Fig.4(a) and Fig.4(b), directions in which a fluid flow is possible (i.e. any flow direction) are symbolized with bold white arrows; and directiopns in which any fluid flow is prevented are symbolized with white arrows each being scored out with a dark line.

In a preferred embodiment as depicted in the Fig3, Fig4(a) and Fig4(b); the edge (30) can be sized and shaped to, in the normal state, prevent the member (3) from spreading in radial directions with regard to the axis (C), and to maintain the inlet side (31) substantially convex, such that the central portion (33) reclines against the opening (41) and thereby exerts an increased amount of mechanical pressure against the opening (41). This can be arranged by provision of an increased thickness to the member throughout its edge (30).

In other words, the member (3) can be arranged in the form of a membrane which has a substantially convex inlet side (31) for more effectively reclining against the opening (41) in the normal state. In this embodiment, the pressure exerted by the member (3) onto the opening (41) in the normal state is inherently higher when compared to a element (5) with a substantially flat inlet side (31). This embodiment thus provides an enhanced sealing of the opening (41) at the normal state. An inlet side perspective view of a member (3) according to such embodiment is shown in Fig.5. The member (3) exemplified in the Fig.5 is provided with a plurality of holes (34) around the central portion (33). Yet, even though the member (3) would be provided with a single hole between the central portion (33) and the edge (30), such member (3) would still function as described above.

The member (3) being provided with a plurality of holes (34) substantially uniformly distributed around the central portion (33) is preferable, since the flexion of such member at transitions between the normal and active states, has an enhanced symmetry around the axis (C). Thus, the fluid flowing through the holes (34) causes a uniform drag force distribution thereto, providing an enhanced service life to the valve (1).

Preferably, a member (3) can be provided with a substantially convex inlet side (31), and further preferably, such member is further provided with a substantially concave outlet side (32); thus providing of a substantially uniform thickness around the central portion (33) and around the through hole(s) (34), thereby having an enhanced flexibility for reversible transitions between the normal state and the activated state of the valve (1).

As also exemplified in Fig.4(a) and Fig.4(b), the shape and size of a section of the opening (41) can be arranged to remain within a projection of the central portion (33). Said section can be substantially perpendicular to a flow direction from the inlet (11) to the outlet (12), and said projection can be substantially parallel to said flow direction. In such embodiment, the hole(s) (34) inherently remain outside said projection, by being positioned around the central portion (33) (Le. between the central portion (34) and the edge (30).

In a further embodiment, the edge (30) of the member (3) can be constrained at the inlet side (31) by a support (4) (which is preferably formed from a substantially rigid/inflexible material), and by a further surface provided in the valve (1) circumferentially supporting the edge (30) outlet side (32). This feature ensures that the member (3) is better secured in place in case of where the pressure at the outlet (12) exceeds the fluid pressure at the inlet (11). The Fig.3, Fig.4(a) and Fig.4(b) visually exemplify such embodiment.

The member (3) can be provided with a lip (35) extended adapted by its shape and size to circumferentially surround a projection of the opening (41) substantially parallel to the axis (C). This embodiment enables exertion of an increased pressure around the opening (41) at the normal state, enhancing the sealing thereof.

The inlet (11) of the valve (1) according to the present invention can be provided with a swabbable connector (20). Swabbable connectors are widely used in the medical technology. Such swabbable connector (20) can be a luer connector, e.g. a male luer connector. Thus, the present invention further provides a reflux-resistant highly compact one-way valve (1) with a luer connector. Said compactness provides a decreased total size and weight to the valve (1), which in use enhances the comfort for a patient, in particular for pediatric use. Furthermore, the compactness has a favorable impact on production costs.

The one-way valve (1) according to the above mentioned preferred embodiment being also swabbable allows an enhanced minimization of a catheter occlusion effect prior to use. Thus a safe and effective anti-microbial barrier can be established and thereby catheter-related bloodstream infections (CRBSI) are effectively avoided.

One or more of the (substantially) rigid materials mentioned in the present description can comprise a thermoset resin.

The member (3) can be substantially formed from an organosilicon polymer. Polydimethyl siloxane (PDMS) can be considered highly suitable for being employed as a material to form the member (3) since it is highly compatible with bodily fluids.

Thus the following objects are achieved by the present invention:
- the abovementioned shortcomings of the prior art are overcome,
- a one way intravenous needle free valve with minimized/eliminated dead volumes is provided,
- a one way intravenous needle free valve with enhanced flow control is provided,
- a one way intravenous needle free valve is provided in which reflux is eliminated.

### List of reference numerals:

- 1: one way intravenous needle free valve, or valve
- 2: shell
- 3: flexible member, or member
- 4: support
- 11: inlet
- 12: outlet
- 20: swabbable connector
- 30: circumferential edge, or edge
- 31: inlet side
- 32: outlet side
- 33: central portion
- 34: hole
- 35: lip
- 41: opening
- C: axis

## Claims

1. A one way intravenous needle free valve (1) including a shell (2) providing a fluid flow path along an axis (C) between an inlet (11) and an outlet (12); a flexible member (3) placed between the inlet (11) and the outlet (12) and constrained throughout a circumferential edge (30) thereof in a liquid-tight manner; an opening (41) with a closed-geometry, in fluid communication with the inlet (11); the opening (41) being aligned with a substantially impermeable central portion (33) provided on the inlet side (31); the member (3) includes one or more holes (34) around the central portion (33), providing a local fluid communication between the inlet side (31) and the outlet side (32); and the member (3) is shaped and sized to recline onto the opening (41) such that the opening (41) is substantially hermetically covered by the central portion (33) when a fluid pressure at the outlet (12) is equal to or greater than a fluid pressure at the inlet (11); the member (3) has an increased thickness around the edge (30),
**characterised in that**
the edge (30) has a size and shape to provide the member (3) with a substantially convex inlet side (31) and a substantially concave outlet side (32).

2. Valve according to the claim 1, wherein the member (3) is provided with a plurality of holes (34) which are substantially uniformly distributed around the central portion (33).

3. Valve according to any one of the claims 1 or 2, wherein the opening (41) has a section with a shape and size arranged to remain within a projection of the central portion (33); and said section is substantially perpendicular to a flow direction from the inlet (11) to the outlet (12); and said projection is substantially parallel to said flow direction.

4. Valve according to any one of the claims 1 to 3, wherein the edge (30) is constrained at the inlet side (31) by a support (4), and by a further surface provided in the valve (1) circumferentially supporting the edge (30) outlet side (32).

5. Valve according to the claim 4, wherein the support (4) is formed from a substantially rigid material.

6. Valve according to the claim 5, wherein the rigid material comprises a thermoset resin.

7. Valve according to any one of the claims 1 to 6, wherein the inlet (11) is provided with a swabbable connector (20).

8. Valve according to the claim 7, wherein said swabbable connector (20) is a luer connector.

9. Valve according to any one of the claims 1 to 8, wherein the member (3) is provided with a lip (35) extended adapted by its shape and size to circumferentially surround a projection of the opening (41) substantially parallel to the axis (C).

10. Valve according to any one of the claims 1 to 9, wherein the member (3) is substantially formed from an organosilicon polymer.

11. Valve according to the claim 10, wherein the member includes PDMS.

## Patentansprüche

1. Intravenöses nadelfreies Einwegventil (1) mit einer Schale (2), die einen Fluidströmungsweg entlang einer Achse (C) zwischen einem Einlass (11) und einem Auslass (12) bildet; ein flexibles Element (3), das zwischen dem Einlass (11) und dem Auslass (12) angeordnet ist und über eine Kante (30) desselben flüssigkeitsdicht eingespannt ist, eine Öffnung (41) mit einer geschlossenen Geometrie, die in Fluidverbindung mit dem Einlass (11) steht; wobei die Öffnung (41) mit einem im Wesentlichen undurchlässigen zentralen Abschnitt (33) ausgerichtet ist, der auf der Einlassseite (31) vorgesehen ist; das Element (3) ein oder mehrere Löcher (34) um den zentralen Abschnitt (33) herum aufweist, die eine lokale Fluidverbindung zwischen der Einlassseite (31) und der Auslassseite (32) herstellen; und das Element (3) so geformt und bemessen ist, dass es sich auf die Öffnung (41) legt, so dass die Öffnung (41) durch den zentralen Abschnitt (33) im Wesentlichen hermetisch abgedeckt ist, wenn ein Fluiddruck am Auslass (12) gleich oder größer ist als ein Fluiddruck am Einlass (11); das Element (3) eine erhöhte Dicke um die Kante (30) herum aufweist,
**dadurch gekennzeichnet,**
**dass** die Kante (30) eine Größe und Form aufweist, um das Element (3) mit einer im Wesentlichen konvexen Einlassseite (31) und einer im Wesentlichen konkaven Auslassseite (32) zu versehen.

2. Ventil nach Anspruch 1, wobei das Element (3) mit einer Vielzahl von Löchern (34) ausgestattet ist, die im Wesentlichen gleichmäßig um den zentralen Abschnitt (33) verteilt sind.

3. Ventil nach einem der Ansprüche 1 oder 2, wobei die Öffnung (41) einen Abschnitt mit einer Form und Größe aufweist, die so angeordnet ist, dass sie innerhalb eines Vorsprungs des zentralen Abschnitts (33) verbleibt; und der Abschnitt im Wesentlichen senkrecht zu einer Strömungsrichtung vom Einlass (11) zum Auslass (12) ist; und der Vorsprung im Wesentlichen parallel zu der Strömungsrichtung ist.

4. Ventil nach einem der Ansprüche 1 bis 3, wobei die Kante (30) an der Einlassseite (31) durch eine Stütze (4) und durch eine weitere im Ventil (1) vorgesehene Fläche, die die Auslassseite (32) der Kante (30) in Umfangsrichtung stützt, eingespannt ist.

5. Ventil nach Anspruch 4, wobei die Stütze (4) aus einem im wesentlichen starren Material gebildet ist.

6. Ventil nach Anspruch 5, wobei das starre Material ein duroplastisches Harz umfasst.

7. Ventil nach einem der Ansprüche 1 bis 6, wobei der Einlass (11) mit einem abwischbaren Anschluss (20) versehen ist.

8. Ventil nach Anspruch 7, wobei der abwischbare Anschluss (20) ein Luer-Anschluss ist.

9. Ventil nach einem der Ansprüche 1 bis 8, wobei das Element (3) mit einer verlängerten Lippe (35) versehen ist, die durch ihre Form und Größe so angepasst ist, dass sie eine Projektion der Öffnung (41) im Wesentlichen parallel zur Achse (C) in Umfangsrichtung umgibt.

10. Ventil nach einem der Ansprüche 1 bis 9, wobei das Element (3) im wesentlichen aus einem siliziumorganischen Polymer gebildet ist.

11. Ventil nach Anspruch 10, wobei das Element PDMS enthält.

## Revendications

1. Valve antiretour exempte d'aiguille intraveineuse (1) comportant une coque (2) fournissant un trajet d'écoulement de fluide le long d'un axe (C) entre une entrée (11) et une sortie (12) ; un élément flexible (3) placé entre l'entrée (11) et la sortie (12) et contraint sur tout un bord circonférentiel (30) de celui-ci d'une manière étanche au liquide ; une ouverture (41) avec une géométrie fermée, en communication fluidique avec l'entrée (11) ; l'ouverture (41) étant alignée avec une partie centrale (33) sensiblement imperméable prévue sur le côté d'entrée (31) ; l'élément (3) comporte un ou plusieurs trous (34) autour de la partie centrale (33), fournissant une communication fluidique locale entre le côté d'entrée (31) et le côté de sortie (32) ; et l'élément (3) est façonné et dimensionné pour s'incliner sur l'ouverture (41) de telle sorte que l'ouverture (41) est sensiblement hermétiquement couverte par la partie centrale (33) lorsqu'une pression de fluide au niveau de la sortie (12) est égale à ou supérieure à une pression de fluide au niveau de l'entrée (11) ; l'élément (3) présente une épaisseur plus élevée autour du bord (30),
**caractérisée en ce que**
le bord (30) présente une taille et forme pour pourvoir l'élément (3) d'un côté d'entrée (31) sensiblement convexe et d'un côté de sortie (32) sensiblement concave.

2. Valve selon la revendication 1, dans laquelle l'élément (3) est doté d'une pluralité de trous (34) qui sont distribués sensiblement uniformément autour de la partie centrale (33).

3. Valve selon l'une quelconque des revendications 1 ou 2, dans laquelle l'ouverture (41) présente une section avec une forme et taille agencées pour rester dans une saillie de la partie centrale (33) ; et ladite section est sensiblement perpendiculaire à une direction d'écoulement à partir de l'entrée (11) vers la sortie (12) ; et ladite saillie est sensiblement parallèle à ladite direction d'écoulement.

4. Valve selon l'une quelconque des revendications 1 à 3, dans laquelle le bord (30) est contraint au niveau du côté d'entrée (31) par un support (4), et par une autre surface prévue dans la valve (1) supportant de manière circonférentielle le côté de sortie (32) du bord (30).

5. Valve selon la revendication 4, dans laquelle le support (4) est formé à partir d'un matériau sensiblement rigide.

6. Valve selon la revendication 5, dans laquelle le matériau rigide comprend une résine thermodurcissable.

7. Valve selon l'une quelconque des revendications 1 à 6, dans laquelle l'entrée (11) est dotée d'un raccord écouvillonnable (20).

8. Valve selon la revendication 7, dans laquelle ledit raccord écouvillonnable (20) est un raccord Luer.

9. Valve selon l'une quelconque des revendications 1 à 8, dans laquelle l'élément (3) est doté d'une lèvre (35) étendue adaptée par ses forme et taille pour entourer de manière circonférentielle une saillie de l'ouverture (41) sensiblement parallèle à l'axe (C).

10. Valve selon l'une quelconque des revendications 1 à 9, dans laquelle l'élément (3) est sensiblement formé à partir d'un polymère organosilicié.

11. Valve selon la revendication 10, dans laquelle l'élément comporte du PDMS.
